# EUROPEAN PATENT SPECIFICATION

(11) **EP 2 591 799 B1**
(45) Date of publication and mention of the grant of the patent: **08.03.2017**
(21) Application number: 11803495.8
(22) Date of filing: 30.06.2011
(51) Int. Cl.: C07K 14/47, A61K 39/00

(54) **CANCER PEPTIDE VACCINE**
KREBSPEPTID-IMPFSTOFF
VACCIN PEPTIDIQUE CONTRE LE CANCER

(30) Priority: 07.07.2010 JP 2010154921
(43) Date of publication of application: 15.05.2013
(73) Proprietor: Green Peptide Co., Ltd., Fukuoka 839-0864 (JP)
(72) Inventor: ITOH, Kyogo, Kurume-shi Fukuoka 830-0011 (JP); YAMADA, Akira, Kurume-shi Fukuoka 830-0011 (JP)
(74) Representative: Vossius & Partner Patentanwälte Rechtsanwälte mbB
(86) International application number: PCT/JP2011/065033
(87) International publication number: WO 2012/005161

(56) References cited:
- EP-A1- 2 196 209
- WO-A1-2007/083763
- WO-A1-2009/038026
- WO-A1-2010/050181
- JP-A- 2005 099 001
- JP-A- 2005 170 799
- JP-A- 2007 145 715
- DATABASE WPI Week 200563 Thomson Scientific, London, GB; AN 2005-619189 XP002723175, & WO 2005/083074 A1 (UNIV KANAZAWA TECHNOLOGY LICENSING ORG) 9 September 2005 (2005-09-09)

### SEQUENCE LISTING

<110> Green Peptide Co., Ltd.
<120> Cancer Peptide Vaccine
<130> 670383
<150> JP 2010-154921
   <151> 2010-07-07
<160> 14
<170> PatentIn version 3.2
<210> 1
   <211> 10
   <212> PRT
   <213> Artificial
<220>
   <223> EGF-R-800
<400> 1
   Asp Tyr Val Arg Glu His Lys Asp Asn Ile
   1               5                   10
<210> 2
   <211> 9
   <212> PRT
   <213> Artificial
<220>
   <223> EZH2-735
<400> 2
   Lys Tyr Val Gly Ile Glu Arg Glu Met
   1               5
<210> 3
   <211> 9
   <212> PRT
   <213> Artificial
<220>
   <223> Lck-208
<400> 3
   His Tyr Thr Asn Ala Ser Asp Gly Leu
   1               5
<210> 4
   <211> 9
   <212> PRT
   <213> Artificial
<220>
   <223> Lck-486
<400> 4
   Thr Phe Asp Tyr Leu Arg Ser Val Leu
   1               5
<210> 5
   <211> 10
   <212> PRT
   <213> Artificial
<220>
   <223> Lck-488
<400> 5
   Asp Tyr Leu Arg Ser Val Leu Glu Asp Phe
   1               5                   10
<210> 6
   <211> 9
   <212> PRT
   <213> Artificial
<220>
   <223> MRP3-503
<400> 6
   Leu Tyr Ala Trp Glu Pro Ser Phe Leu
   1               5
<210> 7
   <211> 10
   <212> PRT
   <213> Artificial
<220>
   <223> MRP3-1293
<400> 7
   Asn Tyr Ser Val Arg Tyr Arg Pro Gly Leu
   1               5                   10
<210> 8
   <211> 9
   <212> PRT
   <213> Artificial
<220>
   <223> PAP-213
<400> 8
   Leu Tyr Cys Glu Ser Val His Asn Phe
   1               5
<210> 9
   <211> 10
   <212> PRT
   <213> Artificial
<220>
   <223> PSA-248
<400> 9
   His Tyr Arg Lys Trp Ile Lys Asp Thr Ile
   1               5                   10
<210> 10
   <211> 9
   <212> PRT
   <213> Artificial
<220>
   <223> PSMA-624
<400> 10
   Thr Tyr Ser Val Ser Phe Asp Ser Leu
   1               5
<210> 11
   <211> 10
   <212> PRT
   <213> Artificial
<220>
   <223> PTH-rP-102
<400> 11
   Arg Tyr Leu Thr Gln Glu Thr Asn Lys Val
   1               5                   10
<210> 12
   <211> 9
   <212> PRT
   <213> Artificial
<220>
   <223> SART2-93
<400> 12
   Asp Tyr Ser Ala Arg Trp Asn Glu Ile
   1               5
<210> 13
   <211> 9
   <212> PRT
   <213> Artificial
<220>
   <223> SART2-161
<400> 13
   Ala Tyr Asp Phe Leu Tyr Asn Tyr Leu
   1               5
<210> 14
   <211> 10
   <212> PRT
   <213> Artificial
<220>
   <223> SART3-109
<400> 14
   Val Tyr Asp Tyr Asn Cys His Val Asp Leu
   1               5                   10

## Description

### Technical Field

The present invention relates to a composition as characterized in the appended claims comprising a group of peptides derived from tumor antigens for use in the immunotherapy of cancer as characterized in the appended claims.

### Background Art

In the world, about 10 million people newly develop cancer annually, and about 6 million of them die. In Japan, the number of patients developing cancer per year is about 600,000 and about 330,000 people or more die of cancer annually and account for about 30% of the total death toll. The major therapeutic approaches conventionally performed for such cancer are surgery, radiotherapy, chemotherapy, and the like. In recent years, antibody drugs targeting molecules such as a receptor expressed by cancer cells have been actively developed and are clinically used. However, these therapeutic approaches temporarily exhibit effect but cannot stop the progression of cancer. Thus, research and development on immunotherapy as a next-generation therapeutic approach having a new action mechanism are carried out.

In the immunotherapy, cell therapy using tumor antigen-presenting cells (dendritic cells) (Provenge from Dendreon, US, or the like) have been ahead worldwide; a preliminary report of pivotal P-III trial results was announced in April 2009; and approval was obtained from the FDA in the US in 2010.

For vaccine therapy using a tumor antigen as a medicinal agent, trials on the prevention of postoperative recurrence of non-small cell cancer using MAGE-A3 antigen discovered by Ludwich Institute and the development of a peptide vaccine against brain tumor (glioblastoma) using malignant EGFRvIII have been performed.

For vaccines intended for the prevention of cancer, Merck &Co., Inc. and GSK Co., Ltd. each have obtained approval for preventive vaccines against cervical cancer.

However, cell therapy as one of the immunotherapies being now ahead has a problem that it can be practically performed only in equipped institutions because of absolutely requiring cells harvested from patients. The peptide vaccine derived from MAGE-A3 or EGFRvIII now under development takes a drug form in which the same antigen is administered to all patients; however, since the protein expression pattern on cancer cells varies for each patient (Non Patent Literature 52), cancer cells in some patients may not express the antigen administered, in which case a sufficient effect cannot probably be expected. In fact, a report is present that such immunotherapy does not have a sufficient efficacy rate (Non Patent Literature 51).

Accordingly, the present inventors have focused attention on the fact that cancer cells in each patient are a heterogeneous population and the antigen causing an immune response to cancer is different for each patient, and have attempted the research and development of the so-called "tailor-made cancer peptide vaccine", for which a plurality of tumor antigen peptides are identified, an immune response to each peptide of the group of peptides is determined for each patient, and the optimal peptide providing an immune response is administered.

For the immunotherapy of cancer, it is considered that an antigen expressed by a cancer cell is presented on the cell surface through a human leukocyte antigen (HLA) molecule and a cytolytic T-lymphocyte (CTL) recognizing it injures the cancer cell to arrest the progression of cancer. The present inventors identified several hundred tumor antigen peptides and conducted intensive studies on a method in which about 80 of these peptides were used to measure the reactivity of CTL present in the peripheral blood of a patient to the peptides, and the optimal peptide was selected and administered (Patent Literatures 1 to 3).

However, in application to a pharmaceutical product, such a method has a problem that it is less easily commercialized because the larger number of the peptides makes the production and development thereof more costly. In addition, on an actual medical site, it also had a problem of making the process from the peptide selection to administration complicated, such as increasing burdens for storing the peptides and controlling the peptides so as to avoid the mix-up thereof. On the other hand, there was also concern that the decreased number of the peptides resulted in the inability to select the optimal peptide in some patients and reduced the effect of the medicinal agent. The peptide selection by the measurement using CTL had problems, such as taking time and the difficulty of measurement in which sufficient reproducibility was guaranteed.

### Citation List

### Patent Literature

Patent Literature 1: WO2005/041982
Patent Literature 2: WO2005/123122
Patent Literature 3: WO03/025569
Patent Literature 4: JP11-318455A
Patent Literature 5: WO00/12701
Patent Literature 6: WO01/011044
Patent Literature 7: WO03/050140
Patent Literature 8: JP2005-162679A
Patent Literature 9: JP2005-170799A
Patent Literature 10: WO2005/071075
Patent Literature 11: JP2007-145715A
Patent Literature 12: WO2009/038026
Patent Literature 13: JP2003-000270A

### Non Patent Literature

Non Patent Literature 1: Nakao M, Shichijo S, Imaizumi T, Inoue Y, Matsunaga K, Yamada A, Kikuchi M, Tsuda N, Ohta K, Takamori S, Yamana H, Fujita H, and Itoh K. Identification of a gene coding for a new squamous cell carcinoma antigen recognized by the CTL. J Immunol. 164:2565-2574, 2000.
Non Patent Literature 2: Yang D, Nakao M, Shichijo S, Sasatomi T, Takasu H, Matsumoto H, Mori K, Hayashi A, Yamana H, Shirouzu K, and Itoh K. Identification of a gene coding for a protein possessing shared tumor epitopes capable of Inducing HLA-A24-restricted cytotoxic T lymphocytes in cancer patients. Cancer Res. 59:4056-4063, 1999.
Non Patent Literature 3: Harashima N, Tanaka K, Sasatomi T, Shimizu K, Miyagi Y, Yamada A, Tamura M, Yamana H, Itoh K, and Shichijo S. Recognition of the Lck tyrosine kinase as a tumor antigen by cytotoxic T lymphocytes of cancer patients with distant metastases. Eur J Immunol. 31:323-332, 2001.
Non Patent Literature 4: Inoue Y, Takaue Y, Takei M, Kato K, Kanai S, Harada Y, Tobisu K, Noguchi M, Kakizoe T, Itoh K, and Wakasugi H. Induction of tumor specific cytotoxic T lymphocytes in prostate cancer using prostatic acid phosphatase derived HLA-A2402 binding peptide. J Urol. 166:1508-1513, 2001.
Non Patent Literature 5: Harada M, Kobayashi K, Matsueda S, Nakagawa M, Noguchi M, and Itoh K. Prostate-specific antigen-derived epitopes capable of inducing cellular and humoral responses in HLA-A24+ prostate cancer patients. Prostate. 57:152-159, 2003.
Non Patent Literature 6: Kobayashi K, Noguchi M, Itoh K, and Harada M. Identification of a prostate-specific membrane antigen-derived peptide capable of eliciting both cellular and humoral immune responses in HLA-A24+ prostate cancer patients. Cancer Sci. 94(7):622-627, 2003. Non Patent Literature 7: Ogata R, Matsueda S, Yao A, Noguchi M, Itoh K, and Harada M. Identification of polycomb group protein enhancer of zeste homolog 2(EZH2)-derived peptides immunogenic in HLA-A24+ prostate cancer patients. Prostate. 60:273-281, 2004.
Non Patent Literature 8: Yao A, Harada M, Matsueda S, Ishihara Y, Shomura H, Noguchi M, Matsuoka K, Hara I, Kamidono S, and Itoh K. Identification of parathyroid hormone-related protein-derived peptides immunogenic in human histocompatibility leukocyte antigen-A24+ prostate cancer patients. Br J Cancer. 91:287-296, 2004.
Non Patent Literature 9: Shomura H, Shichijo S, Komatsu N, Matsueda S, Mine T, Rikimaru T, Sato Y, and Itoh K. Identification of epidermal growth factor receptor-derived peptides recognised by both cellular and humoral immune responses in HLA- A24+ non-small cell lung cancer patients. Eur J Cancer. 40:1776-1786, 2004.
Non Patent Literature 10: Yamada A, Kawano K, Koga M, Matsumoto T, and Itoh K. Multidrug resistance-associated protein 3 is a tumor rejection antigen recognized by HLA-A2402-restricted cytotoxic T lymphocytes. Cancer Res. 61: 6459-6466, 2001.
Non Patent Literature 11: Miyagi Y, Imai N, Sasatomi T, Yamada A, Mine T, Katagiri K, Nakagawa M, Muto A, Okouchi S, Isomoto H, Shirouzu K, Yamana H, and Itoh K. Induction of cellular immune responses to tumor cells and peptides in colorectal cancer patients by vaccination with SART3 peptides. Clin Can Res. 7:3950-3962, 2001.
Non Patent Literature 12: Hida N, Maeda Y, Katagiri K, Takasu H, Harada M, and Itoh K. A simple culture protocol to detect peptide-specific cytotoxic T lymphocyte precursors in the circulation. Cancer Immunol Immunother. 51:219-228, 2002.
Non Patent Literature 13: Komatsu N, Shichijo S, Maeda Y, and Itoh K. Measurement of interferon-γ by high-throughput fluorometric microvolume assay technology (FMAT) system. J Immunol Methods. 263:169-176, 2002.
Non Patent Literature 14: Noguchi M, Kobayashi K, Suetsugu N, Tomiyasu K, Suekane S, Yamada A, Itoh K, and Noda S. Induction of cellular and humoral immune responses to tumor cells and peptides in HLA-A24 positive hormone-refractory prostate cancer patients by peptide vaccination. Prostate. 57: 80-92, 2003.
Non Patent Literature 15: Tanaka S, Harada M, Mine T, Noguchi M, Gohara R, Azuma K, Tamura M, Yamada A, Morinaga A, Nishikori M, Katagiri K, Itoh K, Yamana H, and Hashimoto T. Peptide vaccination for patients with melanoma and other types of cancer based on pre-existing peptide-specific cytotoxic T-lymphocyte precursors in the periphery. J Immunother. 26(4):357-366, 2003.
Non Patent Literature 16: Sato Y, Shomura H, Maeda Y, Mine T, Une Y, Akasaka Y, Kondo M, Takahashi S, Shinohara T, Katagiri K, Sato M, Okada S, Matsui K, Yamada A, Yamana H, Itoh K, and Todo S. Immunological evaluation of peptide vaccination for patients with gastric cancer based on pre-existing cellular response to peptide. Cancer Sci. 94(9):802-808, 2003.
Non Patent Literature 17: Noguchi M, Itoh K, Suekane S, Yao A, Suetsugu N, Katagiri K, Yamada A, Yamana H, and Noda S. Phase I trial of patient-oriented vaccination in HLA-A2-positive patients with metastatic hormone-refractory prostate cancer. Cancer Sci. 95: 77-84, 2004.
Non Patent Literature 18: Harada M, Gohara R, Matsueda S, Muto A, Oda T, Iwamoto Y, and Itoh K. In vivo evidence that peptide vaccination can induce HLA-DR-restricted CD4+ T cells reactive to a class I tumor peptide. J Immunol. 172: 2659-2667, 2004.
Non Patent Literature 19: Tsuda N, Mochizuki K, Harada M, Sukehiro A, Kawano K, Yamada A, Ushijima K, Sugiyama T, Nishida T, Yamana H, Itoh K, and Kamura T. Vaccination with predesignated or evidence-based peptides for patients with recurrent gynecologic cancers. J Immunother. 27: 60-72, 2004.
Non Patent Literature 20: Mine T, Sato Y, Noguchi M, Sasatomi T, Gouhara R, Tsuda N, Tanaka S, Shomura H, Katagiri K, Rikimaru T, Shichijo S, Kamura T, Hashimoto T, Shirouzu K, Yamada A, Todo S, Itoh K, and Yamana H. Humoral responses to peptides correlate with overall survival in advanced cancer patients vaccinated with peptides based on pre-existing, peptide-specific cellular responses. Clin Cancer Res. 10: 929-937, 2004.
Non Patent Literature 21: Sato Y, Maeda Y, Shomura H, Sasatomi T, Takahashi M, Une Y, Kondo M, Shinohara T, Hida N, Katagiri K, Sato K, Sato M, Yamada A, Yamana H, Harada M, Itoh K, and Todo S. A phase I trial of cytotoxic T-lymphocyte precursor-oriented peptide vaccines for colorectal carcinoma patients. Br J Cancer. 90: 1334-1342, 2004.
Non Patent Literature 22: Noguchi M, Itoh K, Suekane S, Morinaga A, Sukehiro A, Suetsugu N, Katagiri K, Yamada A, and Noda S. Immunological monitoring during combination of patient-oriented peptide vaccination and estramustine phosphate in patients with metastatic hormone refractory prostate cancer. Prostate. 60: 32-45, 2004.
Non Patent Literature 23: Mochizuki K, Sato Y, Tsuda N, Shomura H, Sakamoto M, Matsuura K, Ushijima K, Maeda Y, Katagiri K, Yamada A, Todo S, Kamura T, Harada M, and Itoh K. Immunological evaluation of vaccination with pre-designated peptides frequently selected as vaccine candidates in an individualized peptide vaccination regimen. Int J Oncol. 25: 121-131, 2004.
Non Patent Literature 24: Takedatsu H, Okamura T, Yoshimoto K, Harada M, Koga M, Shichijo S, Sata M, and Itoh K. Expression of epithelial cancer-related antigens in hematologic malignancies applicable for peptide-based immunotherapy. J Immunother. 27(4): 289-297, 2004.
Non Patent Literature 25: Komatsu N, Shichijo S, Nakagawa M, and Itoh K. New multiplexed flow cytometric assay to measure anti-peptide antibody: a novel tool for monitoring immune responses to peptides used for immunization. Scand J Clin Lab Invest. 64: 535-545, 2004.
Non Patent Literature 26: Noguchi M, Itoh K, Yao A, Mine T, Yamada A, Obata Y, Furuta M, Harada M, Suekane S, and Matsuoka K. Immunological evaluation of individualized peptide vaccination with a low dose of estramustine for HLA-A24+ HRPC patients. Prostate. 63: 1-12, 2005.
Non Patent Literature 27: Yajima N, Yamanaka R, Mine T, Tsuchiya N, Honma J, Sano M, Kuramoto T, Obata Y, Komatsu N, Arima Y, Yamada A, Shigemori M, Itoh K, and Tanaka R. Immunologic evaluation of personalized peptide vaccination for patients with advanced malignant glioma. Clin Cancer Res. 11(16). 5900-5911, 2005
Non Patent Literature 28: Rosenberg SA, Yang JC, and Restifo NP. Cancer immunotherapy: moving beyond current vaccines. Nat Med. 10(9): 909-915, 2004.
Non Patent Literature 29: Jaeckle KA, Hess KR, Yung WK, Greenberg H, Fine H, Schiff D, Pollack IF, and Kuhn J. Phase II evaluation of temozolomide and 13-cis-retinoic acid for the treatment of recurrent and progressive malignant glioma: a North American brain tumor consortium study. J Clin Oncol. 21(12): 2305-2311, 2003.
Non Patent Literature 30: Prados MD, Yung WK, Fine HA, Greenberg HS, Junck L, Chang SM, Nicholas MK, Robins HI, Mehta MP, Fink KL, Jaeckle KA, Kuhn J, Hess KR, Schold SC Jr., and North American Brain Tumor Consortium study. Phase 2 study of BCNU and temozolomide for recurrent glioblastoma multiforme: North American brain tumor consortium study. Neuro-oncol. 6: 33-37, 2004.
Non Patent Literature 31: Rich J N, Reardon DA, Peery T, Dowell JM, Quinn JA, Penne KL, Wikstrand CJ, Van Duyn LB, Dancey JE, McLendon RE, Koa JC, Stenzel TT, Ahmed Rasheed BK, Tourt-Ushlig SE, Herndon JE 2nd, Vredenburgh JJ, Sampson JH, Friedman AH, Bigner DD, and Friedman HS. Phase II trial of gefitinib in recurrent glioblastoma. J Clin Oncol. 22: 133-142, 2004.
Non Patent Literature 32: Stupp R, Mason WP, van den Bent MJ, Weller-M, Fisher B, Taphoorn MJ, Belanger K, Brandes AA, Marosi C, Bogdahn U, Curschmann J, Janzer RC, Ludwin SK, Gorlia T, Allgeier A, Lacombe D, Cairncross JG, Eisenhauer E, and Mirimanoff RO; European Organisation for Research and Treatment of Cancer Brain Tumor and Radiotherapy Groups; National Cancer Institute of Canada Clinical Trials Group. Radiotherapy plus concomitant and adjuvant temozolomide for glioblastoma. N Engl J Med 352(10): 1036-1038, 2005.
Non Patent Literature 33: Yagoda A, and Petrylak D. Cytotoxic chemotherapy for advanced hormone-resistant prostate cancer. Cancer. 71: 1098-1109, 1993.
Non Patent Literature 34: Scher HI, Mazumadar M, and Kelly WK. Clinical trials in relapsed prostate cancer: Defining the target. J Natl Cancer Inst. 88(22): 1623-1634, 1996.
Non Patent Literature 35: de Voogt HJ, Smith PH, Pavone-Macaluso M, de Pauw M, and Suciu S. Cardiovascular side effects of diethylstilbestrol, cyproterone acetate, medroxyprogesterone acetate and estramustine phosphate used for treatment of advanced prostatic cancer. Results from European organization for research on treatment of cancer trials 30761 and 30762. J Urol. 135: 303-307, 1986.
Non Patent Literature 36: Tannock IF, de Wit R, Berry WR, Horti J, Pluzanska A, Chi KN, Oudard S, Theodore C, James ND, Turesson I, Rosenthal MA, and Eisenberger MA; TAX 327 Investigators. Docetaxel plus prednisone or mitoxantrone plus prednisone for advanced prostate cancer. N Engl J Med. 351: 1502-1512, 2004.
Non Patent Literature 37: Petrylak DP, Tangen CM, Hussain MH, Lara PN Jr, Jones JA, Taplin ME, Burch PA, Berry D, Moinpour C, Kohli M, Benson MC, Small EJ, Raghavan D, and Crawford ED. Docetaxel and estramustine compared with mitoxantrone and prednisone for advanced refractory prostate cancer. N Engl J Med. 351: 1513-1520, 2004.
Non Patent Literature 38: Kojima S, Suzuki H, Akakura K, Shimbo M, Ichikawa T, and Ito H. Alternative antiandrogens to treat prostate cancer relapse after initial hormone therapy. J Urol. 171:679-683. 2004.
Non Patent Literature 39: Salgaller ML, Marincola FM, Cormier JN, and Rosenberg SA. Immunization against epitopes in the human melanoma antigen gp100 following patient immunization with synthetic peptides. Cancer Res. 56:4749-4757, 1996.
Non Patent Literature 40: Rosenberg SA, Yang JC, Schwartzentruber DJ, Hwu P, Marincola FM, Topalian SL, Restifo NP, Dudley ME, Schwarz SL, Spiess PJ, Wunderlich JR, Parkhurst MR, Kawakami Y, Seipp CA, Einhorn JH, and White DE. Immunologic and therapeutic evaluation of a synthetic peptide vaccine for the treatment of patients with metastatic melanoma. Nat Med. 4(3):321-327, 1998.
Non Patent Literature 41: Nestle FO, Alijagic S, Gilliet M, Sun Y, Grabbe S, Dummer R, Burg G, and Schadendorf D. Vaccination of melanoma patients with peptide- or tumor lysate-pulsed dendritic cells. Nat Med. 4(3):328-332, 1998.
Non Patent Literature 42: Marchand M, van Baren N, Weynants P, Brichard V, Dreno B, Tessier MH, Rankin E, Parmiani G, Arienti F, Humblet Y, Bourlond A, Vanwijck R, Lienard D, Beauduin M, Dietrich PY, Russo V, Kerger J, Masucci G, Jager E, De Greve J, Atzpodien J, Brasseur F, Coulie PG, van der Bruggen P, and Boon T. Tumor regressions observed in patients with metastatic melanoma treated with an antigenic peptide encoded by gene MAGE-3 and presented by HLA-A1. Int J Cancer 80:219-230, 1999.
Non Patent Literature 43: Slingluff CL Jr., Yamshchikov G, Neese P, Galavotti H, Eastham S, Engelhard VH, Kittlesen D, Deacon D, Hibbitts S, Grosh WW, Petroni G, Cohen R, Wiernasz C, Patterson JW, Conway BP, and Ross WG. Phase 1 trial of a melanoma vaccine with gp100280-288 peptide and tetanus helper peptide in adjuvant: Immunologic and clinical outcomes. Clin Cancer Res. 7:3012-3024, 2001.
Non Patent Literature 44: Wang F, Bade E, Kuniyoshi C, Spears L, Jeffery G, Marty V, Groshen S, and Weber J. Phase I trial of a MART-1 peptide vaccine with incomplete Freund' s adjuvant for resected high-risk melanoma. Clin Cancer Res. 5:2756-2765, 1999.
Non Patent Literature 45: Jager E, Gnjatic S, Nagata Y, Stockert E, Jager D, Karbach J, Neumann A, Rieckenberg J, Chen YT, Ritter G, Hoffman E, Arand M, Old LJ, and Knuth A. Induction of primary NY-ESO-1 immunity: CD8+T lymphocyte and antibody responses in peptide-vaccinated patients with NY-ESO-1+cancers. Proc Natl Acad Sci USA. 97(22):12198-12203, 2000.
Non Patent Literature 46: Tsugawa T, Kuwashima N, Sato H, Fellows-Mayle WK, Dusak JE, Okada K, Papworth GD, Watkins SC, Gambotto A, Yoshida J, Pollack IF, and Okada H. Sequential delivery of interferon-alpha gene and DCs to intracranial gliomas promotes an effective antitumor response. Gene Ther. 11:1551-8, 2004.
Non Patent Literature 47: The committee of brain tumor registry of Japan: Report of brain tumor registry of Japan (1969-1993) 10th edition. Neurol medico-chirurgica 40 (suppl), 2000.
Non Patent Literature 48: Kitamura T. Necessity of re-evaluation of estramustine phosphate sodium (EMP) as a treatment option for first-line monotherapy in advanced prostate cancer. Int J Urology. 8: 33-36, 2001.
Non Patent Literature 49: Noguchi M, Mine T, Yamada A, Obata Y, Yoshida K, Mizoguchi J, Harada M, Suekane S, Itoh K, and Matuoka K. Combination therapy of personalized peptide vaccination and low-dose estramustine phosphate for metastatic hormone refractory prostate cancer patients: an analysis of prognostic factors in the treatment. Oncol Res. 16:341-349, 2007.
Non Patent Literature 50: Murayama K, Kobayashi T, Imaizumi T, Matsunaga K, Kuramoto T, Shigemori M, Shichijo S, and Itoh K, . Expression of the SART3 tumor-rejection antigen in brain tumors and induction of cytotoxic T lymphocytes by its peptides. J Immunother. :23(5) 511-518, 2000.
Non Patent Literature 51: Rosenberg SA, Yang JC, Restifo NP, Cancer immunotherapy: moving beyond current vaccines. Nat Med. 2004 Sep; 10(9) : 909-15.
Non Patent Literature 52: Homma S, Komohara Y, Harada M, Saya H, Todo S, Itoh K, Noguchi M, Differential levels of human leukocyte antigen-class I, multidrug-resistance 1 and androgen receptor expressions in untreated prostate cancer cells: the robustness of prostate cancer. Oncol Rep. 2007 Aug;18(2):343-6
Non Patent Literature 53: Noguchi M, Kakuma T, Uemura H, Nasu Y, Kumon H, Hirao.Y, Moriya F, Suekane S, Matsuoka K, Komatsu N, Shichijo S, Yamada A, Itoh K, A randomized phase II trial of personalized peptide vaccine plus low dose estramustine phosphate (EMP) versus standard dose EMP in patients with castration resistant prostate cancer, Cancer Immunol Immunother. 2010 Jul;59(7):1001-9. Epub 2010 Feb 10.
Non Patent Literature 54: Noguchi M, Yao A, Harada M, Nakashima O, Komohara Y, Yamada S, Itoh K, Matsuoka K, Immunological evaluation of neoadjuvant peptide vaccination before radical prostatectomy for patients with localized prostate cancer, Prostate. 2007 Jun 15;67(9):933-42.
Non Patent Literature 55: Naito M, Itoh K, Komatsu N, Yamashita Y, Shirakusa T, Yamada A, Moriya F, Ayatuka H, Mohamed ER, Matsuoka K, Noguchi M. Dexamethasone did not suppress immune boosting by personalized peptide vaccination for advanced prostate cancer patients. Prostate. 2008 Dec 1;68(16):1753-62.

### Summary of Invention

### Technical Problem

Thus, an object of the present invention is to provide a tailor-made cancer peptide vaccine enabling the administration of the optimal peptide for each patient, which has overcome the above problems.

### Solution to Problem

The present inventors performed studies for a method for selecting peptides by measuring antibodies to peptides in the peripheral blood of patients and have found that a clinical effect is obtained by selecting peptides inducing high antibody reactivity for each patient before administration. Studies on the selected and administered peptides have enabled us to find an optimal set of peptides which can apply to various patients as a tailor-made cancer peptide vaccine.

Specifically, the present invention provides a composition for use in treating cancer, comprising 8 to 13 peptides of a) to d) below:
a) the peptides of SEQ ID NOS: 4, 5, and 14;
b) 5 peptides selected from SEQ ID NOS: 6, 8, 11, 12, and 13;
c) 0 to 2 peptides selected from SEQ ID NOS: 7 and 9; and
d) 0 to 3 peptides selected from SEQ ID NOS: 1, 3, and 10,
wherein the composition is to be used in the manner that antibodies to the respective peptides in the peripheral blood of a patient are to be measured and 3 or 4 peptides to which antibodies are positive are to be selected in the order of measurement from the highest to the lowest and administered to the patient.

The present invention also provides a method for selecting peptides to be administered for the treatment of cancer, comprising measuring antibodies in the peripheral blood of a patient to 8 to 13 peptides of a) to d) below:
a) the peptides of SEQ ID NOS: 4, 5, and 14;
b) 5 peptides selected from SEQ ID NOS: 6, 8, 11, 12, and 13;
c) 0 to 2 peptides selected from SEQ ID NOS: 7 and 9; and
d) 0 to 3 peptides selected from SEQ ID NOS: 1, 3, and 10,
and selecting 3 or 4 peptides to which antibodies are positive in the order from the highest to the lowest.

### Advantageous Effects of Invention

The composition for use according to the appended claims exerts an excellent clinical effect against cancers such as prostate cancer and brain tumor, especially progressive cancers. The composition for use according to the appended claims is a composition comprising a group of peptides and has an advantage that it can be handled similarly to conventional pharmaceuticals because of not requiring special equipment when used.

### Brief Description of Drawings

[Figure 1] Figure 1 is a graph showing the relationship between the dose of a peptide and the value of IFN-γ in HRPC patients.
[Figure 2] Figure 2 is a graph showing the relationship between the dose of a peptide and the value of FIU in HRPC patients.
[Figure 3] Figure 3 is a graph showing the relationship between the dose of a peptide and the value of IFN-γ in glioblastoma patients.
[Figure 4] Figure 4 is a graph showing the relationship between the dose of a peptide and the value of FIU in glioblastoma patients.
[Figure 5] Figure 5 is a graph showing the relationship between the number of times of peptide administration and the value of IFN-γ in HRPC patients.
[Figure 6] Figure 6 is a graph showing the relationship between the number of times of peptide administration and the value of FIU in HRPC patients.
[Figure 7] Figure 7 is a graph showing the relationship between the number of times of peptide administration and the value of IFN-γ in glioblastoma patients.
[Figure 8] Figure 8 is a graph showing the relationship between the number of times of peptide administration and the value of FIU in glioblastoma patients.
[Figure 9] Figure 9 is a graph showing the relationship between the number of times of administration and the values of IFN-γ for initially selected peptides and reselected peptides in HRPC patients.

### Description of Embodiments

The composition for use according to the appended claims is characterized by the appended claims.

The composition for use according to the appended claims comprises 8 to 13 peptides selected from SEQ ID NOS: 1 and 3 to 14 shown below as defined by the appended claims. These peptides are peptides derived from tumor antigen proteins expressed by cancer cells (Patent Literatures 4 to 13) and recognized by CTL in an HLA class I allele, A-24, restricted manner to induce cytotoxic activity against cancer cells. In other words, the composition for use according to the appended claims is a composition to be applied to HLA-A24-positive cancer patients.

| SEQ ID NO | Name | Amino Acid Sequence |
|---|---|---|
| 1 | EGF-R-800 | Asp-Tyr-Val-Arg-Glu-His-Lys-Asp-Asn-Ile |
| 2 | EZH2-735 | Lys-Tyr-Val-Gly-Ile-Glu-Arg-Glu-Met |
| 3 | Lck-208 | His-Tyr-Thr-Asn-Ala-Ser-Asp-Gly-Leu |
| 4 | Lck-486 | Thr-Phe-Asp-Tyr-Leu-Arg-Ser-Val-Leu |
| 5 | 5 Lck-488 | Asp-Tyr-Leu-Arg-Ser-Val-Leu-Glu-Asp-Phe |
| 6 | MRP3-503 | Leu-Tyr-Ala-Trp-Glu-Pro-Ser-Phe-Leu |
| 7 | MRP3-1293 | Asn-Tyr-Ser-Val-Arg-Tyr-Arg-Pro-Gly-Leu |
| 8 | PAP-213 | Leu-Tyr-Cys-Glu-Ser-Val-His-Asn-Phe |
| 9 | PSA-248 | His-Tyr-Arg-Lys-Trp-Ile-Lys-Asp-Thr-Ile |
| 10 | PSMA-624 | Thr-Tyr-Ser-Val-Ser-Phe-Asp-Ser-Leu |
| 11 | PTH-rP-102 | Arg-Tyr-Leu-Thr-Gln-Glu-Thr-Asn-Lys-Val |
| 12 | SART2-93 | Asp-Tyr-Ser-Ala-Arg-Trp-Asn-Glu-Ile |
| 13 | SART2-161 | Ala-Tyr-Asp-Phe-Leu-Tyr-Asn-Tyr-Leu |
| 14 | SART3-109 | Val-Tyr-Asp-Tyr-Asn-Cys-His-Val-Asp-Leu |

The peptides in the composition for use according to the appended claims can be produced by a conventional method; examples thereof include methods as described in Peptide Synthesis, Interscience, New York, 1966; The Proteins, Vol. 2, Academic Press Inc, New York, 1976; Pepuchido Gosei No Kiso To Jikken (Basis and Experiment for Peptide Synthesis) issued by Maruzen Company Ltd., 1985 [in Japanese]; and Pepuchido Gosei (Peptide Synthesis), Iyakuhin No Kaihatsu Zoku (Development of Pharmaceutical Product (Continued)) vol. 14, issued by Hirokawa Shoten, 1991 [in Japanese] but are not limited to them and known methods are widely available. The purification and recovery of a peptide can be performed by combining gel chromatography, ion column chromatography, affinity chromatography, and the like, or by a known method exemplified by a fractionation means and the like based on difference in solubility using ammonium sulfate, alcohol, and the like. A method can also be used which involves, based on the information on the amino acid sequences of peptides, preparing polyclonal or monoclonal antibodies specific thereto and using the antibodies for specific adsorption and recovery, of course.

In the composition for use according to the appended claims, each peptide is made into the form of a solution before administration to a patient. Thus, the composition for use according to the appended claims preferably comprises peptides formulated so that they can be readily made in the form of a solution before administration, although it may comprise powdered peptides. Formulation methods include a method which involves freeze-drying a solution in which each peptide is dissolved in saline or a 1 to 6 w/v% alkali metal hydrogencarbonate aqueous solution.

As a solvent for dissolving a peptide, saline or a 1 to 6 w/v%, lkali metal hydrogencarbonate aqueous solution may be used, or a mixture of physiological saline and the alkali metal hydrogencarbonate aqueous solution may be used. Examples of the alkali metal hydrogencarbonate include lithium hydrogencarbonate, sodium hydrogencarbonate, and potassium hydrogencarbonate; however, particularly preferred is sodium hydrogencarbonate. The amount of saline or the alkali metal hydrogencarbonate aqueous solution for dissolving the peptide is not particularly limited provided that it is an amount enabling the uniform dissolution of the peptide; however, it is preferably such an amount that the resultant peptide solution has a peptide concentration of 0.4% by mass or less, preferably 0.1 to 0.4% by mass, more preferably 0.3 to 0.4% by mass.

The formulation is preferably performed by dispensing the peptide dissolved in a solvent into a vial before lyophilization. The solvent here is suitably saline; however, for a peptide less easily dissolved in saline, the 1 to 6 w/v% alkali metal hydrogencarbonate aqueous solution may be used.

The composition for use according to the appended claims preferably comprises peptides which have been individually dissolved and freeze-dried one by one. However, it may comprise 2 or more peptides which have been dissolved together in a solvent and freeze-dried or peptides which have been mixed after the dissolution and freeze-drying of each peptide.

The peptides thus formulated may be administered by dissolution in water of a pharmaceutically acceptable purity and such an amount that it is isotonic with the plasma.

The composition for use according to the appended claims specifically comprises 8 to 13 peptides of:
a) the peptides of SEQ ID NOS: 4, 5, and 14;
b) 5 peptides selected from SEQ ID NOS: 6, 8, 11, 12, and 13;
c) 0 to 2 peptides selected from SEQ ID NOS: 7 and 9; and
d) 0 to 3 peptides selected from SEQ ID NOS: 1, 3, and 10.
The number of peptides comprising the composition for use according to the appended claims is not particularly limited provided that it is in the range of 8 to 13; the higher number of the peptides is preferable in that it increases the number of patients and cancer types to which they can be adapted.

In one aspect, the composition for use according to the appended claims comprises 8 to 13 peptides of SEQ ID NOS: 4, 5, 14, 6, 8, 11, 12, and 13 which are selected at a high frequency by antibody testing, 0 to 2 peptides selected from SEQ ID NOS: 7 and 9, and 0 to 3 peptides selected from SEQ ID NOS: 1, 3, and 10.

In another aspect, the composition for use according to the appended claims comprises 8 to 10 peptides of SEQ ID NOS: 4, 5, 14, 6, 8, 11, 12, and 13 and 0 to 2 peptides selected from SEQ ID NOS: 7 and 9.

In still another aspect, the composition for use according to the appended claims comprises 8 peptides of SEQ ID NOS: 4, 5, 14, 6, 8, 11, 12, and 13.

The composition for use according to the appended claims contains peptides derived from antigens which are not specific for certain cancer species, for example, peptides derived from SART-3, SART-2, MRP3 and the like; thus, the intended cancer type is not particularly limited. Examples thereof include prostate cancer, pancreas cancer, colorectal cancer, lung cancer, hematopoietic organ cancer, brain tumor, uterine cancer, cervical cancer, stomach cancer, melanoma, thyroid cancer, liver cancer, and esophagus cancer. The composition for use according to the appended claims is preferably used for prostate cancer and brain tumor.

It is also described herein that when targeting prostate cancer, the composition may comprise 7 peptides of SEQ ID NOS: 4, 5, 14, 6, 8, 11, and 12 or SEQ ID NOS: 4, 5, 14, 6, 8, 11, and 13. It is further described herein that alternatively, it may comprise 6 peptides of SEQ ID NOS: 4, 5, 14, 6, 8, and 11.

When targeting brain tumor, the composition for use according to the appended claims may comprise 8 peptides of SEQ ID NOS: 4, 5, 14, 6, 11, 12, 13, and 7. It is also described herein that when targeting a brain tumor, the composition may comprise 7 peptides of SEQ ID NOS: 4, 5, 14, 6, 11, 12, and 13.

The composition for use according to the appended claims is characterized in that it is used in the manner that antibodies to the peptides comprising the composition for use according to the appended claims present in the peripheral blood of patients are measured to select appropriate peptides for each patient for administration.

An antibody in the peripheral blood of a patient can be measured by a known immunoassay method using an antigen-antibody reaction such as a measuring method using ELISA or multiplex technologies provided by Luminex Corporation. Specifically, peptides comprising the composition for use according to the appended claims are each immobilized on a support, and the antibody in the peripheral blood of a patient, binding to the immobilized peptide is detected and measured through fluorescence or the like. A high measurement thereof indicates high reactivity to the peptide in the patient; thus, the peptide is selected as a peptide to be administered.

When antibody reaction can be measured for multiple peptides in the peripheral blood of a patient, a plurality of peptides is selected in the order of reactivity from highest to lowest. The number of the selected peptides is 3 to 4, more preferably 4. It is considered that the larger number of peptides administered to a patient can induce more species of CTL of the patient and can more efficiently eliminate cancer cells which consist a heterogeneous population. On the other hand, the burden of a patient due to administration is reduced by fewer administered peptides. Thus, in accordance with the invention the number of administered peptides as defined by the appended claims is 3 or 4.

Thus, the present invention relates to a method for selecting peptides administered for the treatment of cancer, comprising measuring antibodies in the peripheral blood of a patient to 8 to 13 peptides of a) to d):
a) the peptides of SEQ ID NOS: 4, 5, and 14;
b) 5 peptides selected from SEQ ID NOS: 6, 8, 11, 12, and 13;
c) 0 to 2 peptides selected from SEQ ID NOS: 7 and 9; and
d) 0 to 3 peptides selected from SEQ ID NOS: 1, 3, and 10
and selecting 3 or 4 peptides to which antibodies are positive in the order of measurement from the highest to the lowest.

In one aspect, the method of the present invention comprises measuring antibodies in the peripheral blood of a patient to 8 to 13 peptides of SEQ ID NOS: 4, 5, 14, 6, 8, 11, 12, and 13, 0 to 2 peptides selected from SEQ ID NOS: 7 and 9, and 0 to 3 peptides selected from SEQ ID NOS: 1, 3, and 10, and selecting 3 or 4 peptides to which antibodies are positive in the order of measurement from the highest to the lowest.

In another aspect, the method of the present invention comprises measuring antibodies in the peripheral blood of a patient to 8 to 10 peptides of SEQ ID NOS: 4, 5, 14, 6, 8, 11, 12, and 13 and 0 to 2 peptides selected from SEQ ID NOS: 7 and 9, and selecting 3 or 4 peptides to which antibodies are positive in the order of measurement from the highest to the lowest.

In still another aspect, the method of the present invention comprises measuring antibodies in the peripheral blood of a patient to 8 peptides of SEQ ID NOS: 4, 5, 14, 6, 8, 11, 12, and 13, and selecting 3 or 4 peptides to which antibodies are positive in the order of measurement from the highest to the lowest.

It is also described herein that when targeting prostate cancer, the selection method of the present invention may comprise measuring antibodies in the peripheral blood of a patient to 7 peptides of SEQ ID NOS: 4, 5, 14, 6, 8, 11, and 12 or SEQ ID NOS: 4, 5, 14, 6, 8, 11, and 13 or to 6 peptides of SEQ ID NOS: 4, 5, 14, 6, 8, and 11 and selecting peptide to which antibodies are positive.

When targeting brain tumor, the selection method of the present invention may comprise measuring antibodies in the peripheral blood of a patient to 8 peptides of SEQ ID NOS: 4, 5, 14, 6, 11, 12, 13, and 7. It is also described herein that when targeting a brain tumor, the selection method of the present invention may comprise measuring antibodies in the peripheral blood of a patient to 7 peptides of SEQ ID NOS: 4, 5, 14, 6, 11, 12, and 13 and selecting peptides to which antibodies are positive.

In the method for selecting peptides according to the present invention, 3 to 4 peptides are selected in the order of measurement obtained by measuring antibodies to the peptides, that is, in the order of reactivity of antibody to the peptide, from highest to lowest. The measurement includes, for example, fluorescence intensity (FIU) when a fluorescent label is used, and colorimetry (OD) measurements. When a standard substance is used for quantitation, the measurement may be converted to an amount of the standard substance.

The peptides thus selected for each patient are each made in the form of a solution as described above and are preferably mixed with an adjuvant or the like for administration. Examples of the adjuvant usable in the present invention include Freund's incomplete adjuvant (for example, ISA-51 or the like, SEPPIC Corporation) or polysaccharides such as pullulan capable of emulsifying a peptide solution to increase the retention of peptide in the administration site, and substances having an immunoenhancing effect, such as Freund's complete adjuvant, BCG, alum, GM-CSF, IL-2, and CpG. Freund's incomplete adjuvant is preferable, among others.

The peptides thus prepared are typically subcutaneously administered to a patient. This is because peptides comprising the composition for use according to the appended claims are rapidly decomposed and cannot sufficiently induce an immunological response, for example, when administered by intravenous injection or the like, and because the peptides subcutaneously administered can efficiently activate CTL having cytotoxic activity since under the skin there are antigen-presenting cells capturing an antigen, presenting it on the cell surface via an HLA molecule, and activating T cells such as CTL and B cells.

The administration site is preferably somewhere around the closest possible lymph node to a cancer lesion from the time of the start of the administration; for example, it is a femoral region for prostate cancer and a dorsal region for brain tumor. It may be another region (such as an abdominal or brachial region) when inflammation or the like occurs at the administration site owing to a side effect of administration, making administration difficult.

The dose of peptides is not particularly limited provided that it is a dose at which the subcutaneous administration thereof is acceptable; however, it is preferably 1 mg or more, more preferably 1 to 5 mg, still more preferably 3 to 5 mg by mass of dry peptide powder for one peptide. A dose of more than 5 mg can also be administered.

The administration frequency of peptides is a frequency at which an immunological response is obtained; for example, it is once per 7 to 28 days, preferably once per 7 to 21 days, more preferably once per 7 to 14 days. The administration frequency may be changed during the period of administration; it is contemplated, for example, that such peptides are administered at a frequency of once per 7 days for from the start to the 6th round of administration, administered at a frequency of once per 14 days depending on patient's status at the 7th round and later, and administered at a longer interval (at a frequency of once per 21 to 28 days) if a sufficient immunological response is obtained. In this respect, "patient's status" means patient's burdens arisen from administration, such as inflammation and acute pain at the administration site.

The number of times of administration of peptides is at least 6, preferably 12 or more, more preferably 18 or more. The upper limit of the number of times of administration is not particularly limited provided that the patient can withstand the administration; however, since peptides have been administered up to 84 times in a clinical trial as described in Examples, administration up to 84 times is possible.

Peptides can be thus selected and are to be administered to a patient to activate CTL against the administered peptides to eliminate cancer cells to provide a clinical effect. It is possible that the selection and administration of peptides may not increase the reactivity of antibodies to the peptides or may not activate CTL against the administered peptides. Thus, considering an increase in the reactivity of the antibodies, peripheral blood is preferably again collected every at least 6 times administration, more preferably every 6 to 12 times administration from the patient to measure the reactivity of antibodies to the respective peptides comprising the composition to reselect peptides showing high reactivity. Such reselection allows the optimal peptides showing high reactivity to be selected for each patient and enables a more excellent immunological response to be obtained.

The number of times of reselection of peptides is not particularly limited; however, the reselection is preferably performed once or more, more preferably twice or more. The reaction of antibodies to administered peptides was sufficiently obtained after 24 times administration from the start of administration and the change of peptides to be administered was not performed in the clinical trial as described in Example, and therefore the reselection may be performed 1 to 4 times, preferably 2 to 4 times when peptides are reselected every 6 to 12 times administration.

The composition for use according to the appended claims may be used in combination with another anti-tumor agent or therapeutic approach depending on a cancer species to be treated. Cancer cells in a patient are a heterogeneous population as described above, in which there are present cells incapable of being completely eliminated by an immunological response and cells resistant to an antitumor-agent, a hormone therapy, or the like; thus, the combined use of the composition for use according to the appended claims and another anti-tumor agent or therapeutic approach can increase clinical effects such as the shrinkage of cancer lesions and the prolongation of survival time. Examples of another anti-tumor agent include alkylating agents, antimetabolites, plant alkaloids, topoisomerase inhibitors, microtubule polymerization inhibitors, and molecular-targeted agents; specific examples thereof include 5-FU, estramustine, docetaxel, temozolomide, cisplatin, Gemzar, and rituximab. Examples of another therapeutic approach include surgery, radiation therapy, hormone therapy (a steroid such as dexamethasone, mitoxantrone, prednisolone, estrogen, or progesterone, or an analog agent such as Leuplin).

When the composition for use according to the appended claims is used in combination with another anti-tumor agent or therapeutic approach, such another anti-tumor agent or therapeutic approach is preferably used in the range not affecting the activation of the hematopoietic system and an immunological response because the composition for use according to the appended claims exerts effect by activating hematopoietic cells such as CTL. For example, the composition for use according to the appended claims may be administered after the recovery of lymphocyte count after administering an anti-tumor agent (to 500/mL or more, preferably 1,000/mL or more, for example); another anti-tumor agent or therapeutic approach may be used after administration of the composition for use according to the appended claims; or another anti-tumor agent or therapeutic approach may be administered in the range not causing decreases in leukocyte count and lymphocyte count during the period of administration of the composition for use according to the appended claims.

For example, when the composition for use according to the appended claims is used in combination with estramustine, the dose of estramustine during the period of administration of the composition for use according to the appended claims is preferably 180 to 280 mg/day because estramustine affects the hematopoietic system. When the composition for use according to the appended claims is used in combination with dexamethasone, the dose of dexamethasone during the period of administration of the composition for use according to the appended claims is preferably 0.5 to 1 mg/day because dexamethasone has the effect of suppressing an immunological response. However, Leuplin as an LH-RH analog can be administered at a usual dose even when Leuplin is administered during the period of administration of the composition for use according to the appended claims, because it less affects the hematopoietic system and the immune system. In this way, such combined use can be made during the period of administration of the composition for use according to the appended claims by proper adjustment depending on an agent.

The present invention will be described below in further detail with reference to Example. However, the invention is not intended to be limited to the described Example.

### Example

Using 14 tumor antigen peptides (SEQ ID NOS: 1 to 14), a clinical trial for the indication of prostate cancer and brain tumor (glioblastoma) was carried out.

### (Patient)

Subjects entering into this clinical trial were 15 patients being HLA-A24 positive and having recurrent prostate cancer (HRPC) resistant to hormone therapy and estramustine and 12 patients having progressive glioblastoma (glioblastoma multiforme) being resistant to initial therapy such as surgery and showing recurrence. The patient characteristics had a performance status of 0 or 1 and a lymphocyte count of 1,000/mL or more for prostate cancer patients and a performance status of 0 to 3 and a lymphocyte count of 500/mL or more for glioblastoma patients.

### (Agent)

In the clinical trial, there were used formulations obtained by dissolving each of powdered peptides of SEQ ID NOS: 1 to 14 (more than 95% purity) in saline or sodium bicarbonate and freeze-drying, and Freund's incomplete adjuvant (ISA-51VG, SEPPIC Corporation).

### (Peptide Selection Test and Reselection Test)

An antibody to a peptide in the peripheral blood of patient was measured using a multiplex technology provided by Luminex Corporation by a method as described in Japanese Patent No. 3960614 using each of the powdered peptides of SEQ ID NOS: 1 to 14. More specifically, a carrier was prepared in which each peptide was immobilized on the surface of micro beads (Luminex Corporation) after dissolving the powdered peptide in DMSO. Subsequently, plasma (heparin blood) obtained by blood collection from the patient was mixed with the immobilized carrier to react the antibody contained in the plasma with the peptide immobilized on the carrier. Then, the immobilized carrier was recovered after reaction; the antibody in the peripheral blood of the patient, bound to the peptide on the support was fluorescently labeled using a biotinylated anti-human antibody (Vector Corporation) and an avidinylated fluorescent dye (Streptavidin-PE, Invitrogen Corporation); and the fluorescence intensity (FIU) thereof was measured.

Based on the FIU thus obtained, 4 peptides were selected for each patient in order of decreasing numerical value of FIU. Antibodies to peptides were again measured using plasma obtained by administering the respective peptides 6 times per course to the patient and collecting blood after the end of each course, and 4 peptides were reselected in decreasing order of obtained the FIU value and they were administered to the patient.

### (Administration of Peptide)

The 4 selected peptides were each made in the form of a solution by adding water for injection, emulsified by mixing with ISA-51VG, and subcutaneously administered to the patient. Patients were divided into a group of administration of 1 mg per 1 peptide on a peptide bulk basis (6 HRPC patients and 6 glioblastoma patients), a group of administration of 3 mg (6 HRPC patients and 6 glioblastoma patients), and a group of administration of 5 mg (3 HRPC patients), and administration was performed, setting 1 course to 6 times administration. In the group of administration of 5 mg, administration was performed in a condition that inflammation reaction at an administration site could be accompanied by reduction to 3 mg or up to 2 times discontinuation. Subsequent analysis was performed by counting one time discontinuation as one time administration and setting 1 course to 6 times administration.

The administration was carried out at a frequency of once per 7 days from the start of administration to after the end of the 1st course and at a frequency of once per 7 days or 14 days by the judgment of a clinical investigator depending on the state of inflammation at an administration site in a patient from the 7th and subsequent administration.

The administration site was a femoral region for HRPC patients and a dorsal region for glioblastoma patients, and the selected peptides were individually administered subcutaneously.

The peptide administration was continued while a patient desired the continuation of the therapy and the progression of disease was not observed in the patient, and terminated when the clinical investigator judged that the disease progression was observed. Specifically, for prostate cancer, the clinical investigator judged that the disease was progressive, for example, when an increase in PSA or new metastasis was observed. For brain tumor, the clinical investigator judged that the disease was progressive, for example, when increased lesions were observed in the determination by diagnostic imaging (a bidirectional measurement or RECIST) or when metastasis was observed.

For HRPC patients, during the period of peptide administration, 3.75 mg/month of Leuplin or 3.6 mg/month of goserelin acetate was administered for the maintenance of the infertile condition and 280 mg/day of estramustine was used in combination.

### (Measurement of CTL Activity)

PBMC was separated from the peripheral blood obtained by blood collection at the start of administration and at the end of each course for lyophilization, and CTL activity was measured using all PBMC by a known method (Hida N, Maeda Y, Katagiri K, Takasu H, Harada M, Itoh K., A simple culture protocol to detect peptide-specific cytotoxic T lymphocyte precursors in circulation. Cancer Immunol Immunotherapy 2002; 51: 219-228) after the end of administration. First, PBMC was cultured in the presence of each administered peptide for 2 weeks, and the PBMC was co-cultured with HLA-A24 positive target cells presenting the administered peptide via HLA molecules. Then, CTL activity was measured by measuring IFN-γ secreted into the culture supernatant by the reaction of CTL in PBMC with the target cells. The co-culture was performed under 4 independent conditions for each peptide; 4 values were obtained for each peptide for each PBMC obtained by each blood collection; and the total of the 4 values was used as a value (pg/mL) of IFN-γ for analysis to be described later.

### (Evaluation of Clinical Effect)

The clinical effect was evaluated as follows.

For HRPC, the survival time from the date of definitive registration for the clinical trial was observed over 3 years, and the median value of the survival time (MST) was calculated. As a result, as shown in Table 1, MST was 23.8 months in 15 cases (as of March 10, 2010). It was 29.4 months in subjects to which 3 mg or more was administered and 22.8 months in those to which 1 mg was administered; MST was longer for the administration of 3 mg or more. According to reports using docetaxel as a chemotherapeutic agent for hormone therapy-resistant prostate cancer (Non Patent Literatures 36 and 37), MST was 17.5 months for the combined use of docetaxel and estramustine and 18.9 months for the combined use of docetaxel and prednisolone; in view of this, it was suggested that the composition had the effect of prolonging the survival time.

**[Table 1]**

| | All groups | 1 mg group | 3 mg group + 5 mg group |
|---|---|---|---|
| MST | 23.8 | 22.8 | 29.4 |
| n | 15 | 6 | 9 |

On the other hand, for brain tumor, an MRI test for lesions were performed every 6 times administration to determine the morphology change of tumor. For the bidirectional measurement, the increase or shrinkage of the lesion was determined by the product of the main diameter of a measurable lesion and the maximum diameter perpendicular thereto, and for the RECIST, the increase or shrinkage of the lesion was determined by the sum of the longest diameters of up to 5 measurable lesions. The results are shown in Table 2. In the table, "PR" indicates the presence of a partial response (a 50% or more decrease in the product or a 30% or more decrease in the sum) for either of the bidirectional measurement method or the RECIST method, i.e., an effect of shrinking the lesions; "NC" indicates the absence of increases in the lesions; and "PD" indicates the presence of disease progression such as an increase in the lesion.

**[Table 2]**

| | PR | NC | PD |
|---|---|---|---|
| All groups | 2 | 5 | 5 |
| 1 mg administration group | 1 | 3 | 2 |
| 3 mg administratino group | 1 | 2 | 3 |

The composition resulted in 2 cases of PR and 5 cases of NC for progressive glioblastoma; thus, it was considered that the composition could suppress the disease progression of glioblastoma.

Side effects in 15 HRPC patients and 12 glioblastoma patients were only mild side effects; it could also be confirmed that the composition was excellent in safety.

### (Analysis for Dose and Immunological Response)

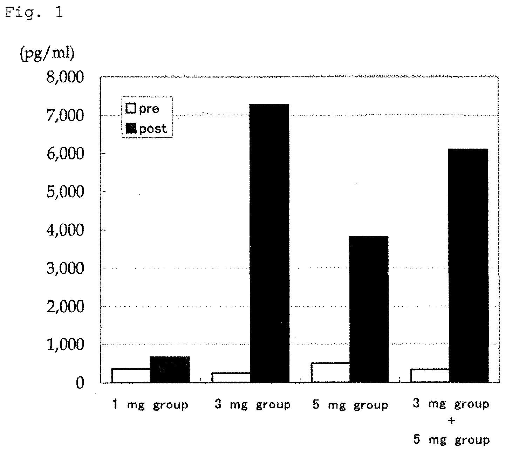

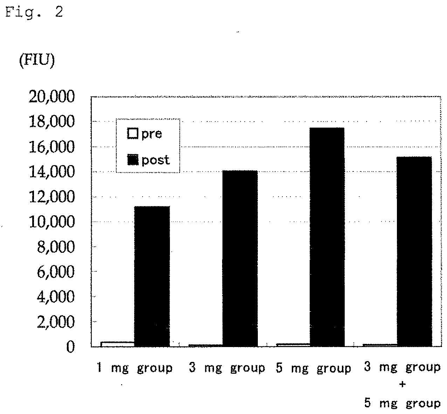

For HRPC patients, the average of IFN-γ values or FIU was calculated in the 1 mg administration group (level I), the 3 mg administration group (level II), and the 5 mg administration group (level III) to analyze the relationship between the dose and the immunological response. The results for IFN-γ values are shown in Table 3 and Figure 1, and the results for FIU are shown in Table 4 and Figure 2.

**[Table 3]**

| Prostate Cancer/CTL | 1 mg group | 3 mg group | 5 mg group | 3 mg group + 5 mg group |
|---|---|---|---|---|
| pre | 368 | 258 | 513 | 343 |
| post | 677 | 7,272* | 3,815 * | 6,087 * |

| | | | | |
|---|---|---|---|---|
| *: p < 0.05, relative to pre-administration (pre) values | | | | |

**[Table 4]**

| Prostate Cancer/Ab | 1 mg group | 3 mg group | 5 mg group | 3 mg group + 5 mg group |
|---|---|---|---|---|
| pre | 387 | 147 | 221 | 171 |
| post | 11,172 * | 14,012* | 17,444* | 15,125* |

| | | | | |
|---|---|---|---|---|
| *: p < 0.05, relative to pre-administration (pre) values | | | | |

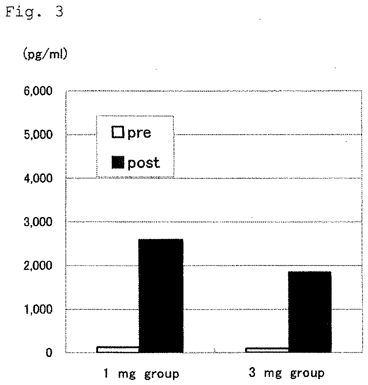

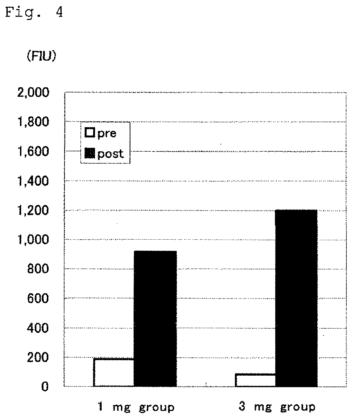

Similarly, for glioblastoma patients, the average of IFN-γ values or FIU was calculated in the 1 mg administration group (level I) and the 3 mg administration group (level II) to analyze the relationship between the dose and the immunological response. The results for IFN-γ values are shown in Table 5 and Figure 3, and the results for FIU are shown in Table 6 and Figure 4.

**[Table 5]**

| Brain Tumor/CTL | 1 mg group | 3 mg group |
|---|---|---|
| pre | 124 | 98 |
| post | 2,594* | 1,845* |

| | | |
|---|---|---|
| *: p < 0.05, relative to pre-administration (pre) values | | |

**[Table 6]**

| Brain Tumor/Ab | 1 mg group | 3 mg group |
|---|---|---|
| pre | 188 | 85 |
| post | 919* | 1,201* |

| | | |
|---|---|---|
| *: p < 0.05, relative to pre-administration (pre) values | | |

For HRPC patients, IFN-γ values were significantly increased after the administration of 3 mg or more (the 3 mg administration group, the 5 mg administration group, or both thereof) compared to those before administration (Table 3). FIU was also significantly increased after administration compared to those before administration in all of the groups (Table 4). On the other hand, for glioblastoma patients, the significant increases in IFN-γ values and FIU by the administration of 1 mg or more were confirmed (Tables 5 and 6).

These results showed that the activation of CTL was obtained by the administration of at least 1 mg, preferably 3 mg or more, more preferably 3 mg to 5 mg. However, for the 5 mg administration, there was present a patient for whom the administration thereof was possible but needs to be performed at a longer interval owing to inflammation at the administration site; thus, considering both of the burden on a patient and the activation of CTL, the peptide dose was considered to be preferably up to 5 mg, more preferably 3 to 5 mg.

### (Analysis for Number of Times of Administration and Immunological Response)

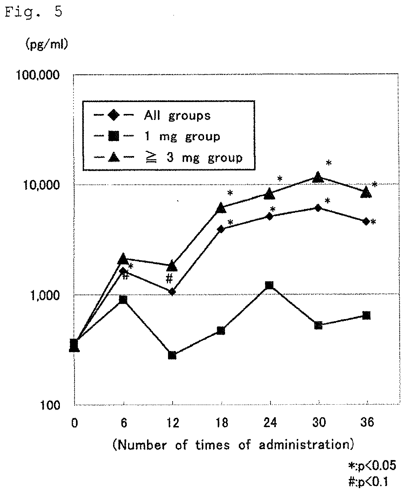

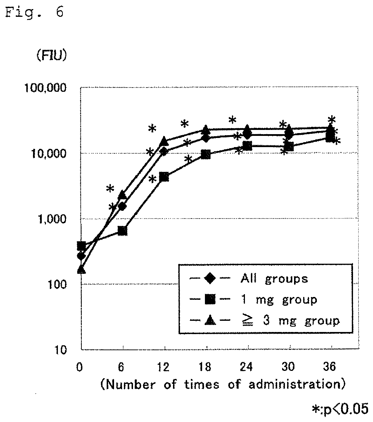

For HRPC patients, the averages of IFN-γ values and FIU obtained from the plasma or PBMC obtained at the end of each course from before administration to the 6th course were calculated in the 1 mg administration group and the group of administration of 3 mg or more to analyze the relationship between the number of times of administration and the immunological response. The results for IFN-γ values are shown in Table 7 and Figure 5, and the results for FIU are shown in Table 8 and Figure 6. The "Number of times of administration" in the table and the figure is the total number of times of administration at the end of each course, and the number of times of administration per course is 6.

**[Table 7]**

| No. of times of administration | 0 | 6 | 12 | 18 | 24 | 30 | 36 |
|---|---|---|---|---|---|---|---|
| All groups | 353 | 1,651 * | 1,065 | 3,912* | 5,139* | 6,097* | 4,586* |
| 1 mg group | 368 | 908 | 284 | 469 | 1,209 | 524 | 640 |
| ≥ 3 mg group | 343 | 2,146^{#} | 1,846^{#} | 6.207* | 8,283* | 11,670* | 8,532* |

| | | | | | | | |
|---|---|---|---|---|---|---|---|
| *: p < 0.05, relative to values for the number of times of administration of "0" #: p < 0.1, relative to values for the number of times of administration of "0" | | | | | | | |

**[Table 8]**

| No. of times of administration | 0 | 6 | 12 | 18 | 24 | 30 | 36 |
|---|---|---|---|---|---|---|---|
| All groups | 271 | 1,542 * | 10.665* | 17,125* | 18.760* | 18.515* | 21.606* |
| 1 mg group | 387 | 652 | 4.349* | 9,497* | 12,702* | 12.358* | 16.966* |
| ≧ 3 mg group | 171 | 2,311* | 15,297* | 22,764* | 23,035* | 22,992 * | 24,137 * |

| | | | | | | | |
|---|---|---|---|---|---|---|---|
| *: p < 0.05, relative to values for the number of times of administration of "0" | | | | | | | |

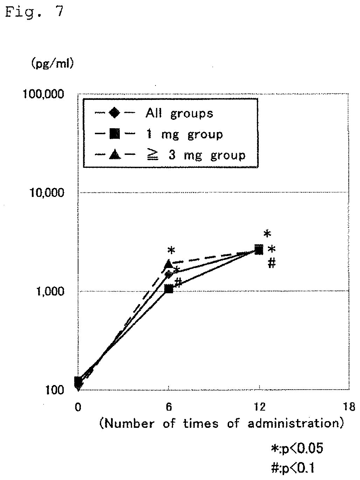

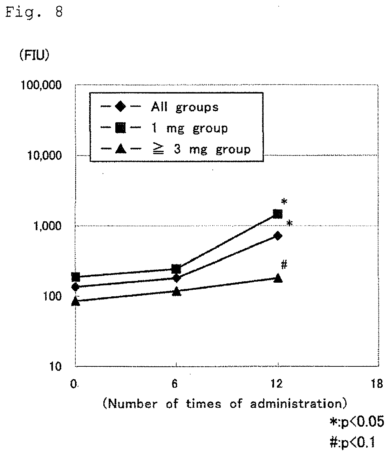

Similarly, for glioblastoma patients, the averages of IFN-γ values and FIU obtained from the plasma or PBMC obtained at the end of each course from before administration to the 2nd course were calculated in the 1 mg administration group (level I) and the group of administration of 3 mg or more (level II) to analyze the relationship between the number of times of administration and the immunological response. The results for IFN-γ values are shown in Table 9 and Figure 7, and the results for FIU are shown in Table 10 and Figure 8. The "Number of times of administration" in the table and the figure is the total number of times of administration at the end of each course, and the number of times of administration per course is 6.

**[Table 9]**

| | 0 | 6 | 12 |
|---|---|---|---|
| All groups | 111 | 1.483* | 2,632* |
| 1 mg group | 124 | 1.060^{#} | 2,679* |
| ≧ 3 mg group | 98 | 1,906* | 2,586^{#} |

| | | | |
|---|---|---|---|
| *: p < 0.05, relative to values for the number of times of administration of "0" #: p < 0.1, relative to values for the number of times of administration of "0" | | | |

**[Table 10]**

| | 0 | 6 | 12 |
|---|---|---|---|
| All groups | 136 | 181 | 715* |
| 1 mg group | 188 | 247 | 1,462* |
| ≧ 3 mg group | 85 | 118 | 181# |

| | | | |
|---|---|---|---|
| *: p < 0.05, relative to values for the number of times of administration of "0" #: p < 0.1, relative to values for the number of times of administration of "0" | | | |

For HRPC patients, CTL was observed to be significantly increased after 18 times administration in the group of administration of 3 mg or more (Table 7). A significant antibody increase was observed after 6 times administration in all groups and after 12 times administration in the 1 mg administration group (Table 8). This showed that CTL could be activated by administering 3 mg or more of each peptide 18 times.

For glioblastoma patients, CTL could be confirmed to be significantly increased from after 6 times administration (Table 9), and a significant antibody increase was confirmed from after 12 times administration (Table 10).

The above results showed that the number of times of peptide administration was at least 6 or more, preferably 12 or more and, for prostate cancer, most preferably 18 or more. The upper limit of the number of times of administration is not particularly limited, and the administration is possible provided that the patient can withstand the administration; however, the administration can be performed at least up to 79 times since the administration was carried out up to 54 times for prostate cancer patients and up to 79 times for glioblastoma patients in this trial.

In addition, the antibody value was shown to reach substantially plateau after 12 times administration (Figure 3). Thus, concerning the frequency of confirming immunological responses to the administered peptides and reselecting peptides, it was also shown that optimal peptides for each patient could be selected by examining an antibody to each peptide every 6 times administration, preferably 6 to 12 times administration.

### (Analysis of Correlation between Antibody and CTL)

Because the CTL activity and the antibody reactivity were shown to be increased depending on the dose and the number of times of administration of peptides, it was analyzed whether or not correlation was present between the increase in the CTL activity and the increase in the antibody activity. The analysis was performed in HRPC patients in whom the number of times of administration was large as the whole.

For the activation of CTL, values obtained by subtracting an IFN-γ value before the peptide administration from IFN-γ values obtained from PBMC at the respective blood collection points were calculated, and the average value (Δ value) of the resultant values was calculated for each patient. On the other hand, for antibodies, because the value of FIU at the start of administration was extremely small compared to those after administration, the average of FIU values obtained at the respective blood collection points was calculated for each patient. Subsequently, the correlation was analyzed between the average values of the Δ values and FIU obtained for each patient. The resultant correlation coefficients are shown in Table 11.

**[Table 11]**

| | All groups | 1mg | 3mg | 5mg | 3mg&5mg |
|---|---|---|---|---|---|
| r | 0.65 | -0.40 | 0.96 | 0.61 | 0.69 |
| n | 15 | 6 | 6 | 3 | 9 |
| p | 0.01 | 0.43 | 0.00 | 0.58 | 0.04 |

A significant correlation was obtained in the 3 mg administration group, the group in which the 3 mg administration group was combined with the 5 mg administration group (the group of administration of 3 mg or more), and all groups, in which CTL was observed to be significantly increased.

The results of Tables 7 to 11 indicated that in the group of administration of 3 mg or more in which a significant response of CTL was obtained, (1) the antibody reactivity was found to be significantly increased from after 6 times administration; (2) the CTL activity was found to have an tendency of increasing from after 6 times administration and significantly increases from after 18 times administration at a later time than the antibody reactivity; and (3) the increase in the antibody reactivity was correlated with the increase in the CTL activity.

In other words, based on the measurements of antibodies exhibiting reaction to respective peptides, peptides could be reselected and administered to efficiently increase CTL against the administered peptides; it was considered that optimal peptides for each patient could be selected and administered by such a selection method.

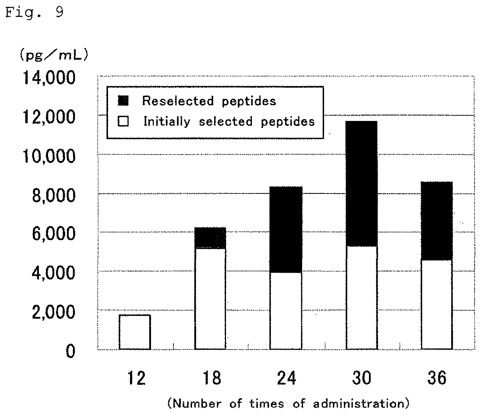

For HRPC patients, the CTL reaction by peptides selected by initial selection and the CTL reaction by reselected and administered peptides were analyzed using IFN-γ values in 7 cases in which the peptide reselection was performed, among the 3 mg administration group and the 5 mg administration group in which significant increases in CTL were observed. The results are shown in Table 12 and Figure 9. Since the initial peptide reselection was performed at the end of the 1st course, the averages of measurements after the reselection (values measured at the end of the 2nd course and later) are shown in the table and the figure. The "Number of times of administration" in the table and the figure is the total number of times of administration at the end of each course, and the number of times of administration per course is 6.

**[Table 12]**

| | | | | | |
|---|---|---|---|---|---|
| Number of times of administration | 12 | 18 | 24 | 30 | 36 |
| Initially selected peptides | 1,736 | 5,163 | 3,982 | 5,291 | 4,604 |
| Reselected peptides | 27 | 1,044 | 4,301 | 6,379 | 3,928 |

Table 12 showed that the proportion of the CTL activation by reselected peptides in the total CTL activation was high after the 24 times administration, which corresponds to after the 18 times administration for peptides administered by reselection after 6 times administration. This showed that the activation of CTL against peptides selected by the peptide reselection contributed significantly to the total CTL activation in each patient. The number of patients in whom the peptide reselection was performed was 6 in 7 after the 6 times administration, 3 in 6 after the 12 times administration, 2 in 5 after the 18 times administration, and 0 after the 24 times administration.

### (Analysis of Immunological Response and Survival Time)

In addition, for HRPC patients, the averages of the Δ values and FIU obtained for each patient were calculated, and their correlation with the survival time was analyzed. The results of the correlation coefficients obtained are shown in Table 13 for the Δ values and in Table 14 for FIU.

**[Table 13]**

| | All groups | 1mg | 3mg | 5mg | 3mg&5mg |
|---|---|---|---|---|---|
| r | 0.29 | -0.69 | 0.92 | 0.51 | 0.53 |
| n | 15 | 6 | 6 | 3 | 9 |
| p | 0.29 | 0.13 | 0.01 | 0.66 | 0.14 |

**[Table 14]**

| | All groups | 1mg | 3mg | 5mg | 3mg&5mg |
|---|---|---|---|---|---|
| r | 0.24 | -0.32 | 0.89 | 0.99 | 0.61 |
| n | 15 | 6 | 6 | 3 | 9 |
| p | 0.39 | 0.54 | 0.02 | 0.07 | 0.08 |

For both Δ value and FIU, the CTL activation or the antibody increase tended to correlate with the survival time in the group of administration of 3 mg or more; particularly, a strong correlation was obtained in the 3 mg administration group. From these results, it was considered that the administration of at least 3 mg or more, preferably 3 to 5 mg of each peptide increased the antibody reactivity and the CTL activity, contributing to the prolongation of the survival time.

### (Analysis of Administered Peptide)

In 15 HRPC patients (402 times administration in total) and 12 glioblastoma patients (198 times administration in total), the frequency of use of each peptide, the number of patients receiving administration, each average value of the measurements of an antibody (FIU) and IFN-γ values for CTL after peptide administration, and the average number of times of administration were calculated. The results are shown in Table 15.

**[Table 15]**

| Peptide | Prostate cancer | | | | | | Brain Tumor | | | | | |
|---|---|---|---|---|---|---|---|---|---|---|---|---|
| | Use Frequency | | Ab | CTL(IFN) | Average number of times of administration | 1FN post average / average no. of times of administration | Use Frequency | | Ab | CTL(IFN) | Average number of times of administration | 1FN post average / average no. of times of administration |
| | Clinical Trial | | Post Average | Post Average | | | Clinical Trial | | Post Average | Post Average | | |
| | Usage Rate | No. of patients | | | | | Usage Rate | No. of patients | | | | |
| GP-101 | 4% | 1 | 16,491 | 129 | 18.0 | 7 | 0% | 0 | - | - | - | - |
| GP-102 | 0% | 0 | - | - | - | - | 0% | 0 | - | - | - | - |
| GP-103 | 3% | 1 | 22 | 100 | 12.0 | 8 | 0% | 0 | - | - | - | - |
| GP-104 | 96% | 15 | 11,538 | 3,629 | 25.2 | 144 | 97% | 12 | 920 | 2,986 | 16.0 | 187 |
| GP-105 | 70% | 12 | 13.096 | 5,274 | 23.0 | 229 | 55% | 8 | 3,969 | 2,706 | 15.0 | 180 |
| GP-106 | 13% | 2 | 20,080 | 22,699 | 27.0 | 841 | 24% | 3 | 297 | 3,134 | 18.0 | 174 |
| GP-107 | 40% | 12 | 2.888 | 370 | 13.5 | 27 | 73% | 8 | 302 | 151 | 18.0 | 8 |
| GP-108 | 72% | 9 | 19,039 | 2,209 | 30.7 | 72 | 0% | 0 | - | - | - | - |
| GP-109 | 30% | 5 | 11,672 | 494 | 22.8 | 22 | 0% | 0 | - | - | - | - |
| GP-110 | 3% | 2 | 310 | 2,190 | 6.0 | 365 | 0% | 0 | - | - | - | - |
| GP-111 | 21% | 4 | 11,125 | 5,576 | 27.0 | 207 | 15% | 4 | 241 | 3,001 | 7.5 | 400 |
| GP-112 | 6% | 4 | 53 | 459 | 6.0 | 76 | 55% | 8 | 1,338 | 1,308 | 14.3 | 92 |
| GP-113 | 10% | 4 | 207 | 600 | 9.0 | 67 | 36% | 5 | 67 | 5,139 | 14.4 | 357 |
| GP-114 | 31% | 11 | 10,557 | 4,592 | 8.4 | 547 | 45% | 9 | 297 | 821 | 10.0 | 82 |

Among SEQ ID NOS: 1 to 14, SEQ ID NO: 2 was not selected in the peptide selection; thus, it was shown that peptides might be selected from a group of 13 peptides of SEQ ID NOS: 1 and 3 to 14. Particularly, it was shown that SEQ ID NOS: 4, 5, and 14 were favorable in the use frequency, the number of patients receiving administration, and the values for antibody and CTL for both prostate cancer and brain tumor and SEQ ID NOS: 6, 8, 11, 12, and 13 were favorable in the use frequency, the number of patients receiving administration, and the value for antibody or CTL for prostate cancer or brain tumor.

On the other hand, SEQ ID NOS: 1, 3, and 10 were low in the use frequency for both prostate cancer and brain tumor; among these, SEQ ID NO: 3 was administered 12 times to 1 HRPC patient but did not increase the reactivity of antibody and CTL and was changed to another peptide later based on the peptide reselection test. On the other hand, SEQ ID NO: 1 was used in 1 HRPC patient but lower in the CTL reactivity than other peptides. In addition, SEQ ID NO: 10 increased the CTL value; however, it was not continually administered although a high value thereof was measured in 1 of 2 cases.

Particularly, SEQ ID NO: 3 did not increase the antibody and CTL compared to SEQ ID NOS: 1 and 10 having a usage frequency comparable thereto; thus, the method of administering peptides by selection using antibodies was considered to provide a sufficient clinical effect even when peptides were selected from a group of 12 peptides, excluding SEQ ID NOS: 2 and 3. It was considered that the exclusion of SEQ ID NO: 3 could be expected to result in a more excellent clinical effect because another peptide more expectable to activate CTL and increase antibody can be selected and administered.

In other words, considering that the continual administration of peptides activated CTL and exerted a life-prolonging effect, it was considered that 12 peptides, excluding SEQ ID NO: 3 from the 13 peptides of SEQ ID NOS 1 and 3 to 14, 11 peptides, further excluding SEQ ID NO: 1 or 10, or 10 peptides, excluding SEQ ID NOS: 1, 3, and 10 from the 13 peptides could be expected to have a sufficient life-prolonging effect. In addition, the exclusion of these peptides results in the administration of peptides more expectable to activate CTL and increase antibodies; thus, considering that the CTL activation and the antibody increase are correlated with the life-prolonging effect, a more excellent clinical effect can be expected by administering other peptides more expected to activate CTL and increase antibodies.

For the 10 peptides of SEQ ID NOS: 4 to 9 and 11 to 14, the IFN-γ value per administration was calculated from the average number of times of administration thereof and IFN-γ values when 1 course was set to 6 times administration of Table 14; as a result, it was considered that SEQ ID NOS: 7 and 9 lowly increased the CTL activity and were weak in the life-prolonging effect by the activation of CTL compared to other peptides. Thus, even 9 peptides, excluding either SEQ ID NO: 7 or 9 from the 10 peptides, or 8 peptides, excluding both of them was considered to provide a life-prolonging effect. In addition, SEQ ID NO: 7 was administered to 12 subjects and SEQ ID NO: 9, to 5 subjects; 1 of the 4 peptides administered in each course was SEQ ID NO: 7 or 9 in all patients. Thus, considering that the CTL activity against these peptides was not sufficiently increased, it was considered that the number of peptides to be administered might be 3 at least.

In addition, among the 8 peptides of SEQ ID NOS: 4 to 6, 8, and 11 to 14, SEQ ID NOS: 12 and 13 had low values of CTL activation and are low in the average number of times of administration compared to the other peptides for HRPC patients; thus, it was considered that they could be excluded on the basis of the same reason as described above. In other words, it was considered that when intended for prostate cancer, the 6 peptides of SEQ ID NOS: 4 to 6, 8, 11, and 14 could be expected to have a sufficient life-prolonging effect. Similarly, because SEQ ID NO: 8 was not used for glioblastoma patients, it was considered that when intended for brain tumor, the 7 peptides of SEQ ID NOS: 4 to 6 and 11 to 14 could be expected to have a life-prolonging effect.

To verify these, HRPC patients were used as subjects to analyze the correlation between the measurement of CTL obtained from each of the sets of 6 to 13 peptides shown in Table 16 and the survival time. The correlation coefficients (R) obtained by the analysis are shown in Table 16. In the table, the "3-peptides concordance percentage" indicates the percentage of patients who have been continually received any 3 of administered peptides through all courses in the 15 patients, and the "antibody positive rate" indicates the percentage of patients for whom antibodies to 3 peptides or more were positive in antibody testing, in the 15 patients.

**[Table 16]**

| No. | Number of peptides | Peptide No. | | All groups (n=15) | 3mg (n=6) | 3mg & 5mg (n=9) | 3-peptides concordance percentage (%) | Antibody positive rate (%) | MST |
|---|---|---|---|---|---|---|---|---|---|
| 1 | 6 | 4,5,14 | 6,8,11 | 0.29 | 0.90 | 0.56 | 60% | 100% | 22.08 |
| 2 | 7 | 4,5,14 | 6,8,11,12 | 0.29 | 0.92 | 0.56 | 67% | 100% | 22.92 |
| 3 | 7 | 4,5,14 | 6,8,11,13 | 0.29 | 0.90 | 0.57 | 73% | 100% | 23.75 |
| 4 | 8 | 4,5,14 | 6,8,11,12,13 | 0.30 | 0.92 | 0.57 | 80% | 100% | 26.60 |
| 5 | 9 | 4,5,14 | 6,7,8,11,12,13 | 0.31 | 0.94 | 0.56 | 87% | 100% | 23.75 |
| 6 | 9 | 4,5,14 | 6,8,9,11,12,13 | 0.32 | 0.91 | 0.57 | 87% | 100% | 23.75 |
| 7 | 10 | 4,5,14 | 6,7,8,9,11,12,13 | 0.32 | 0.93 | 0.57 | 100% | 100% | 23.75 |
| 8 | 11 | 4,5,14 | 1,6,7,8,9,11,12,13 | 0.33 | 0.93 | 0.57 | 100% | 100% | 23.75 |
| 9 | 11 | 4,5,14 | 6,7,8,9,10,11,12,13 | 0.32 | 0.93 | 0.56 | 100% | 100% | 23.75 |
| 10 | 12 | 4,5,14 | 1,6,7,8,9,10,11,12,13 | 0.32 | 0.93 | 0.56 | 100% | 100% | 23.75 |
| 11 | 13 | 4,5,14 | 1,3,6,7,8,9,10,11,12,13 | 0.32 | 0.93 | 0.57 | 100% | 100% | 23.75 |

Table 16 showed that the correlation between the value of CTL and the survival time was not significantly affected even for the set of 6 peptides. It was also shown that 3 peptides or more could be continually administered through all courses in 100% of the patients (15 in 15 patients) for the sets of 10 to 13 peptides, 80% (12 in 15 patients) for the set of 8 peptides, and about 60% for the set of 6 peptides. The MST of subjects were comparable or higher values for the peptide sets of Nos. 3 to 11 but tended to be slightly decreased for the sets of Nos. 1 and 2. From these results, it was considered that an equivalent effect was obtained in most of the subjects by preferably using a set of 7 peptides or more.

Similarly, the 3-peptides concordance percentage and the antibody positive rate were analyzed in 12 glioblastoma patients. The results are shown in Table 17.

**[Table 17]**

| No. | Number of peptides | Peptide No. | | 3-peptides concordance percentage (%) | Antibody positive rate (%) |
|---|---|---|---|---|---|
| 1 | 7 | 4,5,14 | 6,11,12,13 | 100% | 100% |
| 2 | 8 | 4,5,14 | 6,7,11,12,13 | 100% | 100% |
| 3 | 8 | 4,5,14 | 6,8,11,12,13 | 100% | 100% |
| 4 | 9 | 4,5,14 | 6,7,8,11,12,13 | 100% | 100% |
| 5 | 9 | 4,5,14 | 6,8,9,11,12,13 | 100% | 100% |
| 6 | 10 | 4,5,14 | 6,7,8,9,11,12,13 | 100% | 100% |
| 7 | 11 | 4,5,14 | 1,6,7,8,9,11,12,13 | 100% | 100% |
| 8 | 11 | 4,5,14 | 6,7,8,9,10,11,12,13 | 100% | 100% |
| 9 | 12 | 4,5,14 | 1,6,7,8,9,10,11,12,13 | 100% | 100% |
| 10 | 13 | 4,5,14 | 1,3,6,7,8,9,10,11,12,13 | 100% | 100% |

As shown in Table 17, the 3-peptides concordance percentage was 100% (12 in 12 patients) in the glioblastoma patients even for the set of 7 peptides.

In addition, whether differences in the number of times of administration and the dose affected a clinical effect was analyzed by dividing 15 HRPC patients into groups. Two types of analyses were performed: 1) calculating MST by division into the 2 groups of patients receiving the 3 mg-or-more administration 12 times or more and the other patients, and 2) calculating MST by division into the 2 groups of patients receiving the 3 mg-or-more administration 18 times or more and the other patients. The results are shown in Tables 18 and 19.

**[Table 18]**

| | All groups | 1 mg administration or less than 12 times administration | 3 mg-or-more administration 12 times or more |
|---|---|---|---|
| MST | 23.8 | 22.9 | 32.6 |
| n | 15 | 8 | 7 |

**[Table 19]**

| | All groups | 1 mg administration or less than 18 times administration | 3 mg-or-more administration 18 times or more |
|---|---|---|---|
| MST | 23.8 | 21.9 | 32.8 |
| n | 15 | 9 | 6 |

Tables 18 and 19 showed that the group of patients receiving 12 times administration or 18 times administration for which the CTL and the antibody increases could be confirmed tended to have long MST compared to the other groups.

### Industrial Applicability

The composition for use according to the appended claims is a composition comprising a group of peptides derived from tumor antigens and a composition for use in immunotherapy expected as a fourth therapeutic approach after surgery, chemotherapy, and radiation therapy. The composition enables the administration of appropriate peptides for each patient, and is useful because of having a clinical effect of suppressing the progression of difficult-to-treat cancers such as prostate cancer and brain tumor. The composition can also be used in combination with another therapeutic approach.

## Claims

1. A composition for use in treating cancer, comprising 8 to 13 peptides of a) to d) below:
a) the peptides of SEQ ID NOS: 4, 5, and 14;
b) 5 peptides selected from SEQ ID NOS: 6, 8, 11, 12, and 13;
c) 0 to 2 peptides selected from SEQ ID NOS: 7 and 9; and
d) 0 to 3 peptides selected from SEQ ID NOS: 1, 3, and 10,
wherein the composition is to be used in the manner that antibodies to the respective peptides in peripheral blood of a patient are to be measured and 3 or 4 peptides to which antibodies are positive are to be selected in the order of measurement from the highest to the lowest and administered to the patient.

2. The composition for use according to claim 1, wherein the composition comprises 8 to 10 peptides, and wherein the number of the peptides of d) is 0.

3. The composition for use according to claim 1, wherein the composition comprises 8 peptides, and wherein the number of the peptides of each of c) and d) is 0.

4. The composition for use according to any one of claims 1 to 3 for use in treating prostate cancer.

5. The composition for use according to any one of claims 1 to 3, for use in treating a brain tumor.

6. The composition for use according to any of claims 1 to 5, wherein the composition is to be used in the manner that peptides are to be administered in an amount of 3 to 5 mg per 1 peptide.

7. The composition for use according to any of claims 1 to 6, wherein the composition is to be used in the manner that peptides are to be administered at least 18 times or more.

8. The composition for use according to any of claims 1 to 7, wherein the composition is to be used in the manner that peptides are to be administered 6 to 12 times, followed by reselection.

9. The composition for use according to any one of the preceding claims, wherein the composition comprises 12 peptides of SEQ ID NOs: 1 and 4 to 14.

10. A method for selecting peptides to be administered for the treatment of cancer, comprising measuring antibodies in peripheral blood of a patient to 8 to 13 peptides of a) to d) below:
a) the peptides of SEQ ID NOS: 4, 5, and 14;
b) 5 peptides selected from SEQ ID NOS: 6, 8, 11, 12, and 13;
c) 0 to 2 peptides selected from SEQ ID NOS: 7 and 9; and
d) 0 to 3 peptides selected from SEQ ID NOS: 1, 3, and 10;
and selecting 3 or 4 peptides to which antibodies are positive in the order of measurement from the highest to the lowest.

11. The method according to claim 10 comprising measuring antibodies in peripheral blood of a patient to 12 peptides of SEQ ID NOs: 1 and 4 to 14.

## Patentansprüche

1. Zusammensetzung zur Verwendung in der Behandlung von Krebs, umfassend 8 bis 13 Peptide gemäß den nachfolgenden Punkten a) bis d):
a) die Peptide der SEQ ID Nrn: 4, 5 und 14;
b) 5 Peptide ausgewählt aus den SEQ ID Nrn: 6, 8, 11, 12 und 13;
c) 0 bis 2 Peptide ausgewählt aus den SEQ ID Nrn: 7 und 9; und
d) 0 bis 3 Peptide ausgewählt aus den SEQ ID Nrn: 1, 3 und 10,
wobei die Zusammensetzung derart zu verwenden ist, dass Antikörper gegen die jeweiligen Peptide im peripheren Blut eines Patienten zu messen sind und 3 oder 4 Peptide, für die die Antikörper positiv sind, in der Reihenfolge der Messung vom Höchsten zum Niedrigsten auszuwählen und dem Patienten zu verabreichen sind.

2. Zusammensetzung zur Verwendung nach Anspruch 1, wobei die Zusammensetzung 8 bis 10 Peptide umfasst und wobei die Anzahl der Peptide von d) 0 ist.

3. Zusammensetzung zur Verwendung nach Anspruch 1, wobei die Zusammensetzung 8 Peptide umfasst und wobei die Anzahl der Peptide in jedem von c) und d) 0 ist.

4. Zusammensetzung zur Verwendung nach einem der Ansprüche 1 bis 3 zur Verwendung in der Behandlung von Prostatakrebs.

5. Zusammensetzung zur Verwendung nach einem der Ansprüche 1 bis 3 zur Verwendung in der Behandlung eines Gehirntumors.

6. Zusammensetzung zur Verwendung nach einem der Ansprüche 1 bis 5, wobei die Zusammensetzung derart zu verwenden ist, dass Peptide in einer Menge von 3 bis 5 mg pro 1 Peptid zu verabreichen sind.

7. Zusammensetzung zur Verwendung nach einem der Ansprüche 1 bis 6, wobei die Zusammensetzung derart zu verwenden ist, dass Peptide mindestens 18-mal oder häufiger zu verabreichen sind.

8. Zusammensetzung zur Verwendung nach einem der Ansprüche 1 bis 7, wobei die Zusammensetzung derart zu verwenden ist, dass Peptide 6 bis 12-mal zu verabreichen sind, gefolgt von einer Neuauswahl.

9. Zusammensetzung zur Verwendung nach einem der vorherigen Ansprüche, wobei die Zusammensetzung die 12 Peptide der SEQ ID Nrn: 1 und 4 bis 14 umfasst.

10. Verfahren zur Auswahl von Peptiden, die zur Behandlung von Krebs verabreicht werden sollen, umfassend die Messung von Antikörpern in peripherem Blut eines Patienten gegen 8 bis 13 Peptide gemäß den nachfolgenden Punkten a) bis d):
a) die Peptide der SEQ ID Nrn: 4, 5 und 14;
b) 5 Peptide ausgewählt aus den SEQ ID Nrn: 6, 8, 11, 12 und 13;
c) 0 bis 2 Peptide ausgewählt aus den SEQ ID Nrn: 7 und 9; und
d) 0 bis 3 Peptide ausgewählt aus den SEQ ID Nrn: 1, 3 und 10,
und das Auswählen von 3 oder 4 Peptiden, für die die Antikörper positiv sind, in der Reihenfolge der Messung vom Höchsten zum Niedrigsten.

11. Verfahren nach Anspruch 10, umfassend die Messung von Antikörpern gegen die 12 Peptide der SEQ ID Nrn: 1 und 4 bis 14 im peripheren Blut eines Patienten.

## Revendications

1. Composition pour une utilisation dans le traitement du cancer, comprenant 8 à 13 peptides de a) à d) ci-dessous :
a) les peptides de SEQ ID NO : 4, 5, et 14 ;
b) 5 peptides sélectionnés parmi SEQ ID NO : 6, 8, 11, 12, et 13 ;
c) 0 à 2 peptides sélectionnés parmi SEQ ID NO : 7 et 9 ; et
d) 0 à 3 peptides sélectionnés parmi SEQ ID NO : 1, 3, et 10,
dans laquelle la composition doit être utilisée de façon à ce que les anticorps dirigés contre les peptides respectifs dans le sang périphérique d'un patient doivent être mesurés et 3 ou 4 peptides contre lesquels les anticorps sont positifs doivent être sélectionnés dans l'ordre de mesure du plus élevé au plus bas et administrés au patient.

2. Composition pour une utilisation selon la revendication 1, dans laquelle la composition comprend 8 à 10 peptides, et dans laquelle le nombre des peptides de d) est de 0.

3. Composition pour une utilisation selon la revendication 1, dans laquelle la composition comprend 8 peptides, et dans laquelle le nombre des peptides de c) et d) chacun est de 0.

4. Composition pour une utilisation selon l'une quelconque des revendications 1 à 3 pour une utilisation dans le traitement du cancer de la prostate.

5. Composition pour une utilisation selon l'une quelconque des revendications 1 à 3 pour une utilisation dans le traitement d'une tumeur cérébrale.

6. Composition pour une utilisation selon l'une quelconque des revendications 1 à 5, dans laquelle la composition doit être utilisée de façon à ce que les peptides doivent être administrés dans une quantité de 3 à 5 mg pour 1 peptide.

7. Composition pour une utilisation selon l'une quelconque des revendications 1 à 6, dans laquelle la composition doit être utilisée de façon à ce que les peptides doivent être administrés au moins 18 fois ou plus.

8. Composition pour une utilisation selon l'une quelconque des revendications 1 à 7, dans laquelle la composition doit être utilisée de façon à ce que les peptides doivent être administrés 6 à 12 fois, suivie d'une nouvelle sélection.

9. Composition pour une utilisation selon l'une quelconque des revendications précédentes, dans laquelle la composition comprend 12 peptides de SEQ ID NO: 1 et 4 à 14.

10. Méthode de sélection de peptides à administrer pour le traitement du cancer, comprenant la mesure des anticorps dans le sang périphérique d'un patient dirigés contre 8 à 13 peptides de a) à d) ci-dessus
a) les peptides de SEQ ID NO : 4, 5, et 14 ;
b) 5 peptides sélectionnés parmi SEQ ID NO : 6, 8, 11, 12, et 13 ;
c) 0 à 2 peptides sélectionnés parmi SEQ ID NO : 7 et 9 ; et
d) 0 à 3 peptides sélectionnés parmi SEQ ID NO : 1, 3, et 10 ;
et la sélection de 3 ou 4 peptides contre lesquels les anticorps sont positifs dans l'ordre de mesure du plus élevé au plus bas.

11. Méthode selon la revendication 10, comprenant la mesure des anticorps dans le sang périphérique d'un patient dirigés contre 12 peptides de SEQ ID NO : 1 et 4 à 14.
